# EUROPEAN PATENT SPECIFICATION

(11) **EP 4 069 120 B1**
(45) Date of publication and mention of the grant of the patent: **31.01.2024**
(21) Application number: 20819672.5
(22) Date of filing: 01.12.2020
(51) Int. Cl.: A61B 18/14, A61B 1/005, A61B 1/018

(54) **ELECTROSURGICAL INSTRUMENT**
ELEKTROCHIRURGISCHES INSTRUMENT
INSTRUMENT ÉLECTROCHIRURGICAL

(30) Priority: 03.12.2019 GB 201917619
(43) Date of publication of application: 12.10.2022
(73) Proprietor: Creo Medical Limited, Chepstow, Monmouthshire NP16 5UH (GB)
(72) Inventor: HANCOCK, Christopher Paul, Chepstow Monmouthshire NP16 5UH (GB); TURNER, Louis, Chepstow Monmouthshire NP16 5UH (GB); MEADOWCROFT, Simon, Chepstow Monmouthshire NP16 5UH (GB); ULLRICH, George Christian, Bangor Gwynedd LL57 4DB (GB); WEBB, David Edward, Bangor Gwynedd LL57 4DB (GB)
(74) Representative: Mewburn Ellis LLP
(86) International application number: PCT/EP2020/084128
(87) International publication number: WO 2021/110677

(56) References cited:
- WO-A1-2014/184544
- GB-A- 2 569 812
- US-A- 5 833 689

## Description

### FIELD OF THE INVENTION

The invention relates to an electrosurgical instrument for delivering radiofrequency and microwave energy to biological tissue in order to cut and coagulate the tissue. The electrosurgical instrument may be particularly suited for use in flexible endoscopy, e.g. sized to pass through an instrument channel of an endoscope. However, the invention may find applicability in other types of procedure, e.g. rigid laparoscopy or open surgery.

### BACKGROUND TO THE INVENTION

Surgical resection is a means of removing sections of unwanted tissue associated with organs within the human or animal body, such as the liver or the spleen or the bowel. When tissue is cut (divided or transected) small blood vessels called arterioles are damaged or ruptured. Initial bleeding is followed by a coagulation cascade where the blood is turned into a clot in an attempt to plug the bleeding point. During an operation, it is desirable for a patient to lose as little blood as possible, so various devices have been developed in an attempt to provide blood free cutting.

For example, the Hemostatix^{®} Thermal Scalpel System combines a sharp blade with a haemostatic system. The blade is coated with a plastic material and connected to a heating unit which accurately controls the temperature of the blade. The intention is for the heated blade to cauterise the tissue as it is cut.

Other known devices that cut and stop bleeding at the same time do not use a blade. Some devices use radiofrequency (RF) energy to cut and/or coagulate tissue. Other devices (known as harmonic scalpels) use a rapidly vibrating tip to cut tissue.

The method of cutting using RF energy operates using the principle that as an electric current passes through a tissue matrix (aided by the ionic contents of the cells), the impedance to the flow of electrons across the tissue generates heat. When a pure sine wave is applied to the tissue matrix, enough heat is generated within the cells to vaporise the water content of the tissue. There is thus a huge rise in the internal pressure of the cell that cannot be controlled by the cell membrane, resulting in the cell rupturing. When this occurs over a wide area it can be seen that tissue has been transected.

RF coagulation operates by applying a less efficient waveform to the tissue, whereby instead of being vaporised, the cell contents are heated to around 65°C. This dries out the tissue by desiccation and also denatures the proteins in the walls of vessels and the collagen that makes up the cell wall. Denaturing the proteins acts as a stimulus to the coagulation cascade, so clotting is enhanced. At the same time the collagen in the wall is denatured from a rod like molecule to a coil, which causes the vessel to contract and reduce in size, giving the clot an anchor point, and a smaller area to plug.

The application of heat energy to biological tissue is also an effective method of killing cells. For example, the application of microwaves can heat and thus ablate (destroy) biological tissue. This method may in particular be used for the treatment of cancer as the cancerous tissue can be ablated in this way.

WO2014/184544 discloses an electrosurgical instrument according to the preamble of claim 1.

### SUMMARY OF THE INVENTION

The invention is defined by the electrosurgical instrument of claim 1. Embodiments of the invention are defined by the dependent claims. At its most general, the present invention provides an electrosurgical instrument which is capable of simultaneously ablating an area of tissue with microwave energy and performing resection with RF energy. The instrument has a distal bipolar energy delivery tip that has pen-like profile to emit a focussed RF field to facilitate accurate cutting of biological tissue.

According to the invention, there may be provided an electrosurgical instrument comprising: a coaxial transmission line for conveying radiofrequency (RF) energy and microwave energy; an energy delivery tip coupled to a distal end of the coaxial transmission line, wherein the energy delivery tip comprises: a first electrode electrically coupled to an inner conductor of the coaxial transmission line and protruding beyond a distal end of an outer conductor of the coaxial transmission line; a second electrode electrically coupled to the outer conductor of the coaxial transmission line and extending coaxially along a portion of the first electrode; and a dielectric body disposed between the first electrode and second electrode, wherein: the first electrode comprises a projecting nib that protrudes beyond a distal end of the dielectric body; the second electrode and the dielectric body comprise portions that are exposed at the distal end of the energy delivery tip; and the first electrode and second electrode are configured as (i) a bipolar structure for delivering the RF energy conveyed by the coaxial transmission line, and (ii) an antenna for radiating the microwave energy conveyed by the coaxial transmission line.

With the structure above, the instrument of the invention provides focussed delivery of RF energy at the protruding nib. which facilitates accurate cutting by operating the device as a pen to "draw" the cut line. Advantageously, the energy delivery unit is also configured to delivery microwave energy to all rapid coagulation in the event of a bleed. The RF and microwave energy may be applied separately or simultaneously.

In order to achieve an optimal focus of the RF energy, the energy delivery tip has a distally facing end surface that comprises an exposed portion of the dielectric body and an exposed portion of the second electrode arranged concentrically around the projecting nib. Viewed from the front, the instrument may thus resemble a bullseye, with the protruding nib at its centre. The instrument has rotational symmetry around a central longitudinal axis (e.g. the axis of the coaxial transmission line) so that the current effect is uniform regardless of the orientation of the instrument.

The distally facing end surface may be profiled to focus the delivered RF energy at the projecting nib. Profiling the distal end face may also assist in visibility, i.e. by ensuring that the protruding nib can be seen by the operator.

In one example, the distally facing end surface may be conical, i.e. tapered in a linear manner towards the protruding nib. The angle of the conical surface may be selected to assist with visibility and field focussing. In one example, the distally facing end surface may subtend an angle of 45° to a longitudinal axis of the projecting nib.

In another example, the distally facing end surface may be rounded, e.g. dome-like or hemispherical. With the protruding nib, this structure may give the energy delivery tip a bottlenose appearance. The exposed portion of the dielectric body preferably project distally further than the distal end of the exposed portion of the second electrode.

The first electrode may be formed by a distally extending portion of the inner conductor. In other words, the inner conductor may extend unbroken from the coaxial transmission line through the energy delivery tip to form the protruding nib.

However, in other examples, the first electrode may be a separate component from the inner conductor. It may be coupled to the inner conductor via a connector rod. The connector rod may be a further component, or may be formed integrally with the first electrode. An advantage of this configuration is that the dimensions of the connector rod and/or first electrode can be selected independently. This can assist with tuning the impedance of the energy delivery tip for the microwave energy, as discussed below.

The connector rod may comprise a proximal sheath that is secured to an outer surface of a distal end of the inner conductor. The first electrode may thus be connected as an extension of the inner conductor. The connector rod may be connected to the inner conductor by any suitable technique, although a mechanical connection such as crimping may be preferred.

The second electrode may comprise a conductive sleeve having a proximal portion that overlies a distal portion of the outer conductor. The conductive sleeve may be electrically connected and physically secured to the outer conductor in the proximal portion. For example, the conductive sleeve may be secured to the outer conductor by crimping.

The instrument may further comprise an outer insulating jacket, arranged to cover a distal portion of the coaxial transmission line and a proximal portion of the energy delivery tip. The jacket may protect the coaxial transmission line and energy delivery tip and prevent energy from leaking from the structure except at the distal tip.

The antenna may be configured as an impedance transformer to couple the microwave energy into biological tissue. In other words, the energy delivery tip may be configured to transform the impedance of the coaxial transmission line to a typical tissue impedance for the microwave energy. For example, the first electrode, dielectric body and second electrode may have lengths selected to cause the energy delivery tip to operate as a quarter wavelength transformer for the microwave energy.

The instrument may be dimensioned to fit through the instrument channel of a surgical scoping device. For example, the instrument may have a maximum outer diameter equal to or less than 2.0 mm. In some examples, the instrument can be further miniaturised to have a maximum outer diameter equal to or less than 1.0 mm. The protruding nib may have a diameter equal to or less than 0.2 mm. The protruding nib may have a length equal to or less than 1.0 mm.

The term "surgical scoping device" may be used herein to mean any surgical device provided with an insertion tube that is a rigid or flexible (e.g. steerable) conduit that is introduced into a patient's body during an invasive procedure. The insertion tube may include the instrument channel and an optical channel (e.g. for transmitting light to illuminate and/or capture images of a treatment site at the distal end of the insertion tube. The instrument channel may have a diameter suitable for receiving invasive surgical tools. The diameter of the instrument channel may be 5 mm or less. In embodiments of the invention, the surgical scoping device may be an ultrasound-enabled endoscope.

Herein, the term "inner" means radially closer to the centre (e.g. axis) of the instrument channel and/or coaxial cable. The term "outer" means radially further from the centre (axis) of the instrument channel and/or coaxial cable.

The term "conductive" is used herein to mean electrically conductive, unless the context dictates otherwise.

Herein, the terms "proximal" and "distal" refer to the ends of the elongate probe. In use, the proximal end is closer to a generator for providing the RF and/or microwave energy, whereas the distal end is further from the generator.

In this specification "microwave" may be used broadly to indicate a frequency range of 400 MHz to 100 GHz, but preferably the range 1 GHz to 60 GHz. Preferred spot frequencies for microwave EM energy include: 915 MHz, 2.45 GHz, 3.3 GHz, 5.8 GHz, 10 GHz, 14.5 GHz and 24 GHz. 5.8 GHz may be preferred. The device may deliver energy at more than one of these microwave frequencies.

The term "radiofrequency" or "RF" may be used to indicate a frequency between 300 kHz and 400 MHz.

### BRIEF DESCRIPTION OF THE DRAWINGS

Embodiments of the invention are discussed below with reference to the accompanying drawings, in which:
Fig. 1 is a schematic diagram showing an electrosurgical apparatus that is an embodiment of the invention;
Fig. 2 is a schematic sectional view through an instrument cord of an endoscope that can be used with the present invention;
Fig. 3 is a cross-sectional view of a distal end of an electrosurgical instrument that is an embodiment of the invention;
Fig. 4 is a cross-sectional view of a distal end of an electrosurgical instrument that is another embodiment of the invention;
Fig. 5 is a perspective view of the electrosurgical instrument of Fig. 4; and
Fig. 6 is a cross-sectional view of a distal end of an electrosurgical instrument that is another embodiment of the invention.

### DETAILED DESCRIPTION; FURTHER OPTIONS AND PREFERENCES

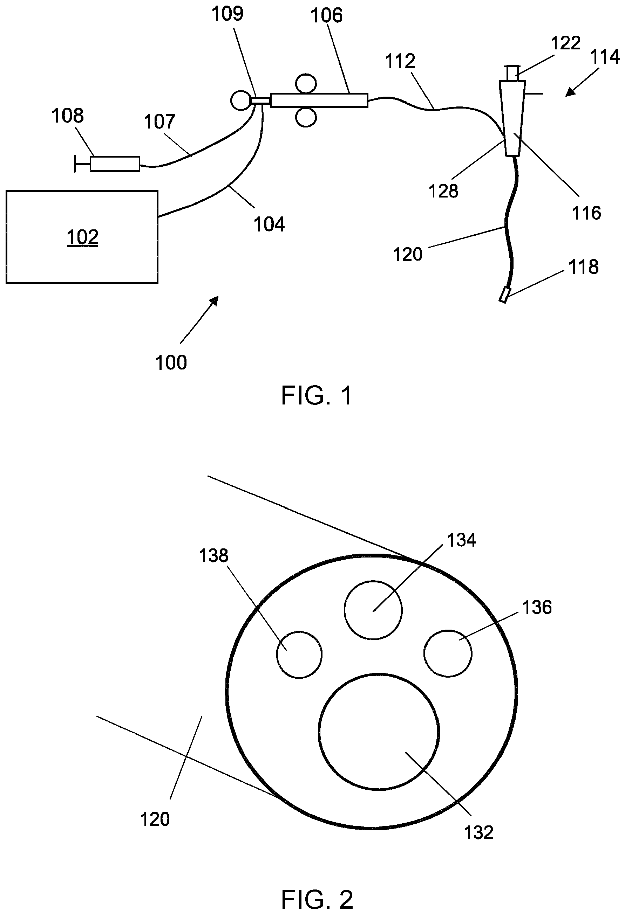

Fig. 1 is a schematic diagram of an electrosurgical apparatus 100 that is capable of supplying radiofrequency energy and microwave energy to the distal end of an invasive electrosurgical instrument. In this example, the apparatus may also be configured to deliver fluid, e.g. cooling fluid, but that is not essential. The system 100 comprises a generator 102 for controllably supplying radiofrequency (RF) and microwave energy. A suitable generator for this purpose is described in WO 2012/076844. The generator may be arranged to monitor reflected signals received back from the instrument in order to determine an appropriate power level for delivery. For example, the generator may be arranged to calculate an impedance seen at the distal end of the instrument in order to determine an optimal delivery power level.

The generator 102 is connected to an interface joint 106 by an interface cable 104. The interface joint 106 is also connected via a fluid flow line 107 to a fluid delivery device 108, such as a syringe. In some examples, the apparatus may be arranged, additionally or alternatively, to aspirate fluid from the treatment site. In this scenario, the fluid flow line 107 may convey fluid away from the interface joint 106 to a suitable collector (not shown). The aspiration mechanism may be connected at a proximal end of the fluid flow line 107.

If needed, the interface joint 106 can house an instrument control mechanism that is operable by sliding a trigger, e.g. to control longitudinal (back and forth) movement of one or more control wires or push rods (not shown). If there is a plurality of control wires, there may be multiple sliding triggers on the interface joint to provide full control. The function of the interface joint 106 is to combine the inputs from the generator 102, fluid delivery device 108 and instrument control mechanism into a single flexible shaft 112, which extends from the distal end of the interface joint 106.

The flexible shaft 112 is insertable through the entire length of an instrument channel (also known as a working channel) of a surgical scoping device 114, which in embodiment of the present invention may comprise an endoscope.

The surgical scoping device 114 comprises a body 116 having a number of input ports and an output port from which an instrument cord 120 extends. The instrument cord 120 comprises an outer jacket which surrounds a plurality of lumens. The plurality of lumens convey various things from the body 116 to a distal end of the instrument cord 120. One of the plurality of lumens is the instrument channel discussed above. Other lumens may include a channel for conveying optical radiation, e.g. to provide illumination at the distal end or to gather images from the distal end. The body 116 may include a eye piece 122 for viewing the distal end.

The flexible shaft 112 has a distal assembly 118 (not drawn to scale in Fig. 1) that is shaped to pass through the instrument channel of the surgical scoping device 114 and protrude (e.g. inside the patient) at the distal end of the instrument cord.

The distal end assembly 118 may be any of the electrosurgical instruments discussed below. The distal end assembly 118 may be particularly designed for use with conventional endoscopes. For example, a maximum outer diameter of the distal end assembly 118 may be equal to or less than 2.0 mm, e.g. less than 1.9 mm (and more preferably less than 1.5 mm) and the length of the flexible shaft can be equal to or greater than 1.2 m. In other example, the structure may be configured for use in even smaller spaces. For example, the maximum outer diameter of the distal end assembly 118 may be equal to or less than 1.0 mm.

The body 116 includes a power input port 128 for connecting to the flexible shaft 112. As explained below, a proximal portion of the flexible shaft may comprise a conventional coaxial cable capable of conveying the radiofrequency and microwave energy from the generator 102 to the distal assembly 118. Coaxial cables that are physically capable of fitting down the instrument channel of an endoscope are available with the following outer diameters: 1.19 mm (0.047"), 1.35 mm (0.053"), 1.40 mm (0.055"), 1.60 mm (0.063"), 1.78 mm (0.070"). Custom-sized coaxial cables having even smaller diameters, e.g. 0.8 mm or less, may also be used.

As discussed above, it is desirable to be able to control the position of at least the distal end of the instrument cord 120. The body 116 may include a control actuator that is mechanically coupled to the distal end of the instrument cord 120 by one or more control wires (not shown), which extend through the instrument cord 120. The control wires may travel within the instrument channel or within their own dedicated channels. The control actuator may be a lever or rotatable knob, or any other known catheter manipulation device. The manipulation of the instrument cord 120 may be softwareassisted, e.g. using a virtual three-dimensional map assembled from computer tomography (CT) images.

Fig. 2 is a view down the axis of the instrument cord 120. In this embodiment there are four lumens within the instrument cord 120. The largest lumen is the instrument channel 132. The other lumens may comprise a pair of illumination channels 136, 138 and a camera channel 134. The invention is however not limited to this configuration. For example, there may be other lumens, e.g. for control wires or fluid delivery or suction.

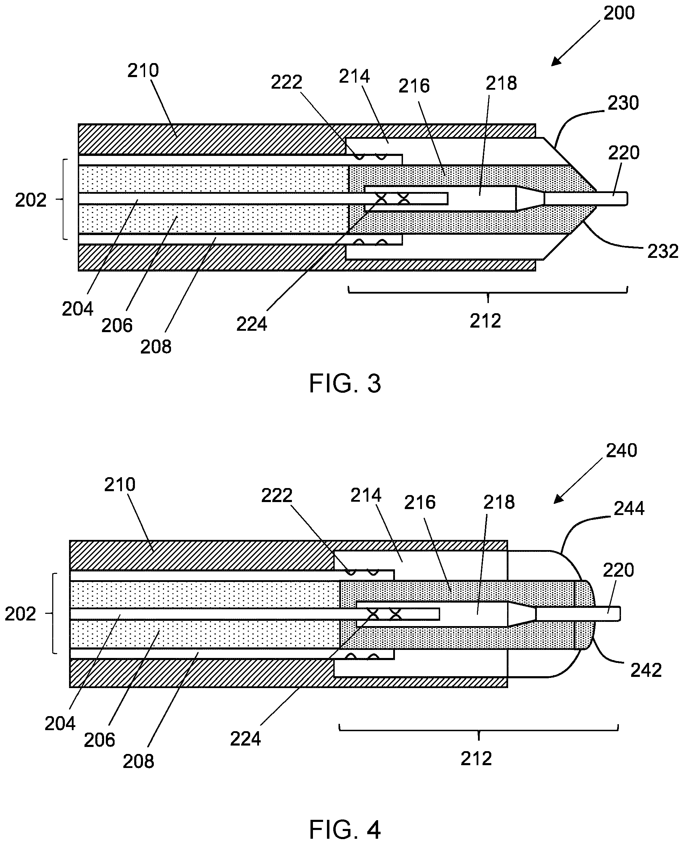

Fig. 3 is a cross-sectional view of a distal end of an electrosurgical instrument 200 that is an embodiment of the invention. The instrument 200 is a generally cylindrical elongate member comprising a flexible coaxial transmission line 202 and a distal energy delivery tip 212. The flexible coaxial transmission line 202 may be a coaxial cable that extends back (e.g. through the instrument channel of a surgical scoping device) to a generator. The coaxial transmission line 202 may be configured to convey radiofrequency (RF) energy and microwave energy, either separately or simultaneously. As explained in more detail below, the distal energy delivery tip 212 may be configured to provide a bipolar element for focussed delivery of RF energy for tissue cutting and coagulation. The distal energy delivery tip 212 may be further configured as an antenna to radiate microwave energy into tissue for coagulation or ablation.

The coaxial transmission line 202 comprises an inner (centre) conductor 204 that is separated from a concentrically arranged outer conductor 208 by a dielectric (electrically insulating) layer 206. An outer surface of the outer conductor 208 is covered by a jacket 210, which providing protection and electrically insulates the outer conductor 208.

A distal end of the coaxial transmission line 202 is connected to the distal energy delivery tip 212. The distal energy delivery tip 212 comprises a dielectric body 216 that extends in a longitudinal direction towards a distal end of the instrument. The longitudinal direction is aligned with the axis of the coaxial cable at the distal end thereof. The dielectric body 216 may be generally cylindrical, and may have an outer diameter that is less than the outer diameter of the coaxial transmission line 202. The dielectric body 216 may be made of the same or a different material to the dielectric layer 206 in the coaxial transmission line 202.

The dielectric body 216 has a hollow longitudinally extending passage running therethrough. The passage may be machined to have appropriate dimensions. At a proximal end, the passage in the dielectric body 216 receives a portion of the inner conductor 204 that extends beyond a distal end of the dielectric layer 206. The inner conductor 204 is electrically coupled to a first electrode 220. The first electrode 220 comprises a rod element that includes a distal portion disposed in the passage of the dielectric body 216 and a proximal portion that protrudes (is exposed at) the distalmost end of the energy delivery tip 212. In this example, the first electrode 220 is electrically (and physically) coupled to the inner conductor 204 by a connector rod 218. The connector rod 218 may be made from an electrically conductive material, e.g. the same material as the inner conductor 204 and/or first electrode 220. The connector rod 218 may have a proximal sleeve part that is secured (e.g. via crimping 224) to a distal part of the inner conductor 204. The first electrode 220 may be integrally formed with the connector rod 218, or may be a separate component that is secured to it.

In practice, the distal energy delivery tip 212 may be manufactured by the following steps:
stripping the dielectric layer 206 and outer conductor 208 from a distal length of the inner conductor 204;
securing the connector rod 218 to the exposed inner conductor 204;
forming, e.g. by wrapping, moulding or the like, the dielectric body 216 around the connector rod.

The energy delivery tip 212 further comprises a second electrode 214, which comprises a conductive sleeve mounted around the dielectric body 216. The conductive sleeve is electrically coupled to the outer conductor 208 of the coaxial transmission line. In this example, a proximal portion of the conductive sleeve is both electrically and physically coupled to a distal portion of the outer conductor 208 via crimping 222.

The jacket 210, which is made from an insulating material extends beyond the coaxial transmission line 202 to cover a portion of the conductive sleeve. However, the jacket 210 stops short of the distal end of the energy delivery tip 212, whereby a distal end portion 230 of the second electrode 214 is exposed.

The distal end of the energy delivery tip 212 therefore resembles a bullseye, comprising: a central projecting nib that is part of the first electrode 220, an exposed portion 232 of the dielectric body 216, and an exposed portion 230 of the second electrode 214 that is separated from the first electrode by the dielectric body 216. The distalmost end of the projecting nib 220 may be rounded, e.g. to prevent snagging on biological tissue in used.

This structure provides a bipolar structure for delivering RF energy. The first electrode 220 and second electrode 214 form active and return poles for the bipolar structure. The bullseye configuration acts to generate a preferential energy flow along the distalmost surface, with an increased energy density at and around the central nib. Such an energy distribution is advantageous for cutting. The instrument may be operated like a pen, because the cutting effect occurs preferentially at the projecting nib. The focussing of the RF energy may occur because the conductive surface area of the projecting nib 220 is less than the surface area of the exposed portion 230. The focussed energy distribution may mean that cutting starts from the projecting nib. The device is therefore intuitive to use.

The distal end of the energy delivery tip 212 may be profiled in a manner that facilitates energy delivery or operation. For example, in Fig. 3 the bullseye has a pointed (e.g. conical) profile, in which the exposed portion 230 of the second electrode 214 and the exposed portion 232 of the dielectric body 216 slope towards the projecting nib 220. The angle of the slope may preferably be in the range 30-60°, preferably 45°.

In addition to delivery RF energy to cut biological tissue, the distal energy delivery tip 212 can be configured as a microwave antenna to deliver microwave energy for coagulation. The relative dimensions of the connector rod 218 and first electrode 220, the dielectric body 216 and second electrode 214 can be selected to ensure that the energy delivery tip 212 has an impedance suitable for coupling microwave energy into biological tissue. In one example, the energy delivery tip 212 may be configured as a quarter wave transformer at the frequency of microwave energy conveying by the coaxial transmission line 202. This configuration operates to facilitate coupling of microwave energy into tissue.

The structure may have a size that is suitable for insertion through the instrument channel of a surgical scoping device, e.g. an endoscope or the like. For example, the coaxial transmission line 202 may be a coaxial cable having an outer diameter of 1.6 mm. The second electrode 214 may have a maximum outer diameter of 2.0 mm. The radial gap between an inner surface of the second electrode 214 and the first electrode 220 (or connector rod 218), i.e. the minimum radial thickness of the dielectric body 216 between the first and second electrodes, may be 0.4 mm. The projecting nib 220 may have a maximum diameter of 0.2 mm.

The instrument may be capable of further miniaturisation. For example, the coaxial cable may have an outer diameter of 0.8 mm, such that the whole device can fit through a passage having a diameter of 1.0 mm.

Fig. 4 is a cross-sectional view of a distal end of an electrosurgical instrument 240 that is another embodiment of the invention. Features in common with Fig. 3 are given the same reference number and are not described again.

The electrosurgical instrument 240 of Fig. 4 differs from Fig. 3 in that the profile of the energy delivery tip 212 has a bottlenose shape. This is defined by the exposed end surface 244 of the second electrode having a rounded, e.g. dome-like distal end, which is curved round to meet a rounded exposed portion 242 of the dielectric body 216. The exposed portion 242 of the dielectric body 216 extends distally beyond a distal end of the exposed portion 244 of the second electrode 214.

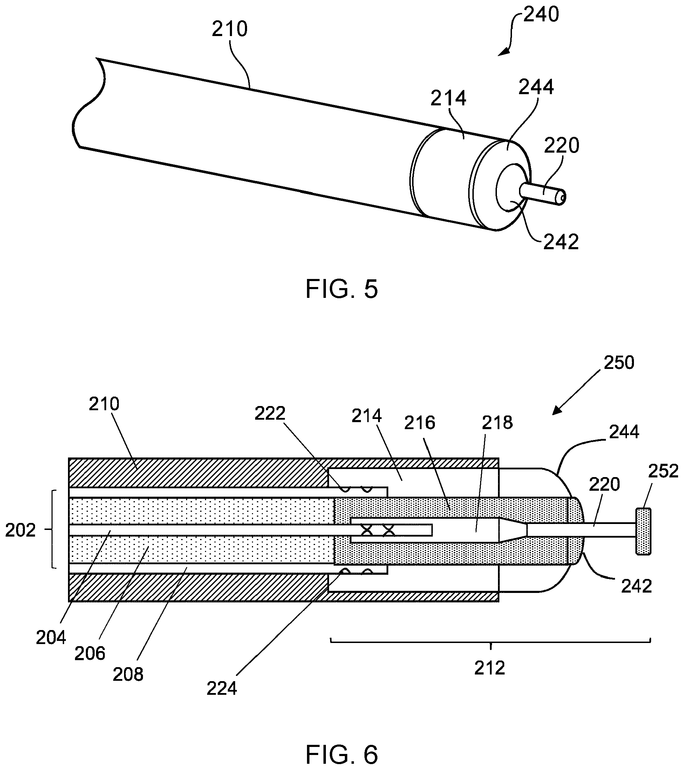

Fig. 5 shows a perspective view of the electrosurgical instrument 240 of Fig. 4.

Fig. 6 is a cross-sectional view of a distal end of an electrosurgical instrument 250 that is another embodiment of the invention. Features in common with Fig. 4 are given the same reference number and are not described again.

The electrosurgical instrument 240 of Fig. 6 differs from Fig. 4 in that the distalmost tip of the projecting nib 220 has an insulating cap 252 formed thereon. The insulating cap 252 may assist in shaping the RF field between the exposed portion 244 of the second electrode 214 and an exposed portion of the projecting nib 220. For example, it may inhibit the RF energy from taking a path that lies beyond the distalmost end of the instrument. The insulating cap 252 may also provide a smooth (e.g. rounded) surface that avoids unwanted tissue damage as the instrument navigates to the treatment site.

## Claims

1. An electrosurgical instrument comprising:
a coaxial transmission line for conveying radiofrequency (RF) energy and microwave energy;
an energy delivery tip coupled to a distal end of the coaxial transmission line, wherein the energy delivery tip comprises:
a first electrode electrically coupled to an inner conductor of the coaxial transmission line and protruding beyond a distal end of an outer conductor of the coaxial transmission line;
a second electrode electrically coupled to the outer conductor of the coaxial transmission line and extending coaxially along a portion of the first electrode; and
a dielectric body disposed between the first electrode and second electrode,
wherein:
the first electrode comprises a projecting nib that protrudes beyond a distal end of the dielectric body;
the second electrode and the dielectric body comprise portions that are exposed at the distal end of the energy delivery tip; and
the first electrode and second electrode are configured as (i) a bipolar structure for delivering the RF energy conveyed by the coaxial transmission line, and (ii) an antenna for radiating the microwave energy conveyed by the coaxial transmission line, **characterised in that** the energy delivery tip has a distally facing end surface that comprises an exposed portion of the dielectric body and an exposed portion of the second electrode arranged concentrically around the projecting nib, such that the electrosurgical instrument has rotational symmetry around a central longitudinal axis of the coaxial transmission line.

2. An electrosurgical instrument according to claim 1, wherein the distally facing end surface is profiled to focus the delivered RF energy at the projecting nib.

3. An electrosurgical instrument according to claim 1 or 2, wherein the distally facing end surface is conical.

4. An electrosurgical instrument according to claim 2, wherein the distally facing end surface subtends an angle of 45° to a longitudinal axis of the projecting nib.

5. An electrosurgical instrument according to claim 1 or 2, wherein the distally facing end surface is rounded.

6. An electrosurgical instrument according to any preceding claim, wherein the first electrode is formed by a distally extending portion of the inner conductor.

7. An electrosurgical instrument according to any one of claims 1 to 5, wherein the first electrode is coupled to the inner conductor via a connector rod.

8. An electrosurgical instrument according to claim 7, wherein the connector rod comprises a proximal sheath that is secured to an outer surface of the inner conductor.

9. An electrosurgical instrument according to claim 7 or 8, wherein the connector rod is connected to the inner conductor by crimping.

10. An electrosurgical instrument according to any preceding claim, wherein the second electrode comprises a conductive sleeve having a proximal portion that overlies a distal portion of the outer conductor.

11. An electrosurgical instrument according to claim 10, wherein the conductive sleeve is secured to the outer conductor by crimping.

12. An electrosurgical instrument according to any preceding claim further comprising an outer insulating jacket, arranged to cover a distal portion of the coaxial transmission line and a proximal portion of the energy delivery tip.

13. An electrosurgical instrument according to any preceding claim, wherein the antenna is configured as an impedance transformer to couple the microwave energy into biological tissue.

14. An electrosurgical instrument according to claim 13, wherein the first electrode, dielectric body and second electrode have lengths selected to cause the energy delivery tip to operate as a quarter wavelength transformer for the microwave energy.

## Patentansprüche

1. Elektrochirurgisches Instrument, das Folgendes umfasst:
eine koaxiale Übertragungsleitung zum Übertragen von Hochfrequenz-(HF-)Energie und Mikrowellenenergie;
eine Energiezufuhrspitze, die mit einem distalen Ende der koaxialen Übertragungsleitung gekoppelt ist, wobei die Energiezufuhrspitze Folgendes umfasst:
eine erste Elektrode, die elektrisch mit einem Innenleiter der koaxialen Übertragungsleitung gekoppelt ist und über ein distales Ende eines Außenleiters der koaxialen Übertragungsleitung hinausragt;
eine zweite Elektrode, die elektrisch mit dem Außenleiter der koaxialen Übertragungsleitung gekoppelt ist und sich koaxial entlang eines Abschnitts der ersten Elektrode erstreckt; und
einen dielektrischen Körper, der zwischen der ersten Elektrode und der zweiten Elektrode angeordnet ist,
wobei:
die erste Elektrode eine vorstehende Nase umfasst, die über ein distales Ende des dielektrischen Körpers hinausragt;
die zweite Elektrode und der dielektrische Körper Abschnitte umfassen, die am distalen Ende der Energiezufuhrspitze freigelegt sind; und
die erste Elektrode und die zweite Elektrode (i) als bipolare Struktur zur Zufuhr der durch die koaxiale Übertragungsleitung übertragenen HF-Energie und (ii) als Antenne zum Ausstrahlen der durch die koaxiale Übertragungsleitung übertragenen Mikrowellenenergie ausgelegt sind, **dadurch gekennzeichnet, dass** die Energiezufuhrspitze eine in distale Richtung gewandte Endfläche aufweist, die einen freigelegten Abschnitt des dielektrischen Körpers und einen freigelegten Abschnitt der zweiten Elektrode, die konzentrisch um die vorstehende Nase angeordnet sind, umfasst, sodass das elektrochirurgische Instrument rotationssymmetrisch um eine Längsmittelachse der koaxialen Übertragungsleitung angeordnet ist.

2. Elektrochirurgisches Instrument nach Anspruch 1,
wobei die in distale Richtung gewandte Endfläche profiliert ist, um die zugeführte HF-Energie auf der vorstehenden Nase zu konzentrieren.

3. Elektrochirurgisches Instrument nach Anspruch 1 oder 2, wobei die in distale Richtung gewandte Endfläche konisch ist.

4. Elektrochirurgisches Instrument nach Anspruch 2, wobei die in distale Richtung gewandte Endfläche im Winkel von 45 ° zu einer Längsachse der vorstehenden Nase steht.

5. Elektrochirurgisches Instrument nach Anspruch 1 oder 2, wobei die in distale Richtung gewandte Endfläche gerundet ist.

6. Elektrochirurgisches Instrument nach einem der vorangegangenen Ansprüche, wobei die erste Elektrode durch einen sich distal erstreckenden Abschnitt des Innenleiters gebildet ist.

7. Elektrochirurgisches Instrument nach einem der Ansprüche 1 bis 5, wobei die erste Elektrode über einen Verbinderstab mit dem Innenleiter gekoppelt ist.

8. Elektrochirurgisches Instrument nach Anspruch 7, wobei der Verbinderstab eine proximale Ummantelung umfasst, die auf einer Außenfläche des Innenleiters fixiert ist.

9. Elektrochirurgisches Instrument nach Anspruch 7 oder 8, wobei der Verbinderstab durch Crimpen mit dem Innenleiter verbunden ist.

10. Elektrochirurgisches Instrument nach einem der vorangegangenen Ansprüche, wobei die zweite Elektrode eine leitfähige Ummantelung mit einem proximalen Abschnitt aufweist, der einen distalen Abschnitt des Außenleiters überlagert.

11. Elektrochirurgisches Instrument nach Anspruch 10, wobei die leitfähige Ummantelung durch Crimpen auf dem Außenleiter fixiert ist.

12. Elektrochirurgisches Instrument nach einem der vorangegangenen Ansprüche, ferner umfassend eine Isolierhülle, die angeordnet ist, um einen distalen Abschnitt der koaxialen Übertragungsleitung und einen proximalen Abschnitt der Energiezufuhrspitze zu bedecken.

13. Elektrochirurgisches Instrument nach einem der vorangegangenen Ansprüche, wobei die Antenne als Impedanzwandler zum Koppeln der Mikrowellenenergie in biologisches Gewebe ausgelegt ist.

14. Elektrochirurgisches Instrument nach Anspruch 13, wobei die erste Elektrode, der dielektrische Körper und die zweite Elektrode Längen aufweisen, die ausgewählt sind, um zu bewirken, dass die Energiezufuhrspitze als Viertelwellenlängenwandler in Bezug auf die Mikrowellenenergie arbeitet.

## Revendications

1. Instrument électrochirurgical, comprenant :
une ligne de transmission coaxiale pour transporter de l'énergie radiofréquence (RF) et de l'énergie hyperfréquence ;
une pointe de distribution d'énergie couplée à une extrémité distale de la ligne de transmission coaxiale, dans lequel la pointe de distribution d'énergie comprend :
une première électrode couplée électriquement à un conducteur interne de la ligne de transmission coaxiale et faisant saillie au-delà d'une extrémité distale d'un conducteur externe de la ligne de transmission coaxiale ;
une seconde électrode couplée électriquement au conducteur externe de la ligne de transmission coaxiale et s'étendant coaxialement le long d'une partie de la première électrode ; et
un corps diélectrique disposé entre la première électrode et la seconde électrode,
dans lequel :
la première électrode comprend un ergot de projection qui fait saillie au-delà d'une extrémité distale du corps diélectrique ;
la seconde électrode et le corps diélectrique comprennent des parties qui sont exposées au niveau de l'extrémité distale de la pointe de distribution d'énergie ; et
la première électrode et la seconde électrode sont configurées sous la forme (i) d'une structure bipolaire pour distribuer l'énergie RF transportée par la ligne de transmission coaxiale, et (ii) d'une antenne pour rayonner l'énergie hyperfréquence transportée par la ligne de transmission coaxiale, **caractérisé en ce que** la pointe de distribution d'énergie présente une surface d'extrémité orientée distalement qui comprend une partie exposée du corps diélectrique et une partie exposée de la seconde électrode agencée concentriquement autour de l'ergot de projection, de sorte que l'instrument électrochirurgical présente une symétrie de rotation autour d'un axe longitudinal central de la ligne de transmission coaxiale.

2. Instrument électrochirurgical selon la revendication 1, dans lequel la surface d'extrémité orientée distalement est profilée pour focaliser l'énergie RF distribuée au niveau de l'ergot de projection.

3. Instrument électrochirurgical selon la revendication 1 ou 2, dans lequel la surface d'extrémité orientée distalement est conique.

4. Instrument électrochirurgical selon la revendication 2, dans lequel la surface d'extrémité orientée distalement sous-tend un angle de 45° par rapport à un axe longitudinal de l'ergot de projection.

5. Instrument électrochirurgical selon la revendication 1 ou 2, dans lequel la surface d'extrémité orientée distalement est arrondie.

6. Instrument électrochirurgical selon l'une quelconque des revendications précédentes, dans lequel la première électrode est formée par une partie s'étendant de manière distale du conducteur interne.

7. Instrument électrochirurgical selon l'une quelconque des revendications 1 à 5, dans lequel la première électrode est couplée au conducteur interne via une tige de connecteur.

8. Instrument électrochirurgical selon la revendication 7, dans lequel la tige de connecteur comprend une gaine proximale qui est fixée à une surface externe du conducteur interne.

9. Instrument électrochirurgical selon la revendication 7 ou 8, dans lequel la tige de connecteur est connectée au conducteur interne par sertissage.

10. Instrument électrochirurgical selon l'une quelconque des revendications précédentes, dans lequel la seconde électrode comprend un manchon conducteur présentant une partie proximale qui recouvre une partie distale du conducteur externe.

11. Instrument électrochirurgical selon la revendication 10, dans lequel le manchon conducteur est fixé au conducteur extérieur par sertissage.

12. Instrument chirurgical selon l'une quelconque des revendications précédentes, comprenant en outre une enveloppe isolante externe, agencée pour recouvrir une partie distale de la ligne de transmission coaxiale et une partie proximale de la pointe de distribution d'énergie.

13. Instrument électrochirurgical selon l'une quelconque des revendications précédentes, dans lequel l'antenne est configurée sous la forme d'un transformateur d'impédance pour coupler l'énergie hyperfréquence dans un tissu biologique.

14. Instrument électrochirurgical selon la revendication 13, dans lequel la première électrode, le corps diélectrique et la seconde électrode présentent des longueurs sélectionnées pour amener la pointe de distribution d'énergie à fonctionner comme un transformateur de quart de longueur d'onde pour l'énergie hyperfréquence.
